(19) Europäisches Patentamt
European Patent Office
Office européen des brevets

(11) Veröffentlichungsnummer: **0 329 011**
**A1**

# (12) EUROPÄISCHE PATENTANMELDUNG

(21) Anmeldenummer: **89102204.8**

(22) Anmeldetag: **09.02.89**

(51) Int. Cl.4: **C07D 209/08 , C07D 235/08 , A01N 43/38 , A01N 43/52**

(30) Priorität: **18.02.88 DE 3805059**

(43) Veröffentlichungstag der Anmeldung: **23.08.89 Patentblatt 89/34**

(84) Benannte Vertragsstaaten: **BE CH DE ES FR GB IT LI NL**

(71) Anmelder: **BAYER AG**

**D-5090 Leverkusen 1 Bayerwerk(DE)**

(72) Erfinder: **Jelich, Klaus, Dr.**
**Pahlkestrasse 5**
**D-5600 Wuppertal 1(DE)**
Erfinder: **Krämer, Wolfgang, Dr.**
**Rosenkranz 25**
**D-5093 Burscheid 2(DE)**
Erfinder: **Dutzmann, Stefan, Dr.**
**Leinenweberweg 33**
**D-4000 Düsseldorf 13(DE)**

## (54) Heterocyclisch substituierte Acrylsäureester.

(57) Heterocyclisch substituierte Acrylsäureester der Formel (I),

$$R^1\text{-O-CH} = \overset{\displaystyle A\\ |}{C}\text{-COOR}^2 \qquad (I)$$

in welcher

$R^1$, $R^2$ und A die in der Beschreibung gegebenen Bedeutungen haben, deren geometrische Isomere und Isomerengemische und die Verwendung der Verbindungen zur Bekämpfung von Schadorganismen, Außerdem neue 3-Hydroxyacrylsäureester.

Die heterocyclisch substituierten Acrylsäureester sind durch die Formel (I) definiert. Sie können nach Analogieverfahren hergestellt werden, z. B. aus geeigneten 3-Hydroxyacrylsäureestern mit geeigneten Alkylierungsmitteln oder aus geeigneten heterocyclisch substituierten Essigsäureestern, geeigneten Ameisensäureestern und geeigneten Alkylierungsmitteln.

Die neuen 3-Hydroxyacrylsäureester sind durch die Formel (IIa) definiert und können ebenfalls nach Analogieverfahren hergestellt werden, z.B. aus geeigneten Indolylessigsäureestern und geeigneten Ameisensäureestern.

EP 0 329 011 A1

## Heterocyclisch substituierte Acrylsäureester

Die Erfindung betrifft neue heterocyclisch substituierte Acrylsäureester, mehrere Verfahren zu ihrer Herstellung sowie ihre Verwendung in Schädlingsbekämpfungsmitteln und neue Ausgangsprodukte.

Es ist bekannt, daß bestimmte heterocyclisch substituierte Alkoxyacrylsäureester, wie beispielsweise die Verbindung 3-Methoxy-2-(2-benzoylpyrrol-1-yl)-acrylsäuremethylester gute fungizide Eigenschaften besitzen (vgl. z.B. EP 206 523).

Die Wirksamkeit dieser vorbekannten Verbindungen ist jedoch insbesondere bei niedrigen Aufwendmengen und Konzentrationen nicht in allen Anwendungsgebieten völlig zufriedenstellend.

Es wurden neue heterocyclisch substituierte Acrylsäureester der allgemeinen Formel (I),

$$R^1\text{-O-CH}=\overset{\overset{\displaystyle A}{|}}{C}\text{-COOR}^2 \qquad \text{(I)}$$

in welcher

A für einen gegebenenfalls substituierten 1-Benzimidazolylrest oder für einen gegebenenfalls substituierten 1-Indolylrest steht und

$R^1$ und $R^2$ unabhängig voneinander jeweils für Alkyl, Alkenyl, Alkinyl, Halogenalkyl, Alkoxyalkyl oder Alkylthioalkyl stehen,

gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen heterocyclisch substituierten Acrylsäureester der allgemeinen Formel (I),

$$R^1\text{-O-CH}=\overset{\overset{\displaystyle A}{|}}{C}\text{-COOR}^2 \qquad \text{(I)}$$

in welcher

A für einen gegebenenfalls substituierten 1-Benzimidazolyl oder für einen gegebenenfalls substituierten 1-Indolylrest steht und

$R^1$ und $R^2$ unabhängig voneinander jeweils für Alkyl , Alkenyl, Alkinyl, Halogenalkyl, Alkoxyalkyl oder Alkylthioalkyl stehen,

erhält, wenn man

(a) 3-Hydroxyacrylsäureester oder deren Alkalimetallsalze der Formel (II),

$$\text{M-O-CH}=\overset{\overset{\displaystyle A}{|}}{C}\text{-COOR}^2 \qquad \text{(II)}$$

in welcher

A und $R^2$ die oben angegebene Bedeutung haben und

M für Wasserstoff oder für ein Alkalimetallkation steht,

mit Alkylierungsmitteln der Formel (III),

$$R^1\text{-E} \qquad \text{(III)}$$

in welcher

$R^1$ die oben angegebene Bedeutung hat und

E für eine elektronenanziehende Abgangsgruppe steht,

gegebenenfalls in Gegenwart eines Verdünnungsmittels und gegebenenfalls in Gegenwart eines Reaktionshilfsmittels umsetzt oder wenn man

(b) heterocyclisch substituierte Essigsäureester der Formel (IV),

$$\text{A-CH}_2\text{-COOR}^2 \qquad \text{(IV)}$$

in welcher

A und $R^2$ die oben angegebene Bedeutung haben,

mit Ameisensäureestern der Formel (V),

$$R^3\text{-O-}\overset{\overset{\displaystyle O}{\|}}{C}\text{-H} \qquad \text{(V)}$$

in welcher

$R^3$ für Alkyl steht,

und anschließend mit Alkylierungsmitteln der Formel (III),

$$R^1\text{-E} \qquad \text{(III)}$$

in welcher

$R^1$ die oben angegebene Bedeutung hat und

E für eine elektronenanziehende Abgangsgruppe steht, gegebenenfalls in Gegenwart eines Verdünnungsmittels und gegebenenfalls in Gegenwart eines Reaktionshilfsmittels umsetzt.

Schließlich wurde gefunden, daß die neuen heterocyclisch substituierten Acrylsäureester der Formel (I) eine gute Wirksamkeit gegen Schädlinge besitzen.

Überraschenderweise zeigen die erfindungsgemäßen heterocyclisch substituierten Acrylsäureester der allgemeinen Formel (I) eine erheblich höhere fungizide Wirksamkeit als die aus dem Stand der Technik bekannten heterocyclisch, substituierten Alkoxyacrylsäureester, wie beispielsweise die Verbindung 3-Methoxy-2-(2-benzoylpyrrol-1-yl)-acrylsäuremethylester, welches chemische und wirkungsmäßig naheliegende Verbindungen sind.

Die erfindungsgemäßen heterocyclisch substituierten Acrylsäureester sind durch die Formel (I) allgemein definiert. Bevorzugt sind Verbindungen der Formel (I), bei welchen

A für einen jeweils gegebenenfalls einfach oder mehrfach, gleich oder verschieden substituierten 1-Benzimidazolylrest oder 1-Indolylrest steht, wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen oder jeweils gegebenenfalls im Arylteil einfach oder mehrfach, gleich oder verschieden durch Halogen, Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil und

$R^1$und $R^2$ unabhängig voneinander jeweils für jeweils geradkettiges oder verzweigtes Alkyl mit 1 bis 6 Kohlenstoffatomen, Alkenyl mit 2 bis 6 Kohlenstoffatomen, Alkinyl mit 2 bis 6 Kohlenstoffatomen, Alkoxyalkyl oder Alkylthioalkyl mit jeweils 1 bis 4 Kohlenstoffatomen in den einzelnen Alkylteilen oder Halogenalkyl mit 1 bis 4 Kohlenstoffatomen und 1 bis 9 gleichen oder verschiedenen Halogenatomen stehen.

Besonders bevorzugt sind Verbindungen der Formel (I), bei welchen

A für einen jeweils gegebenenfalls ein- bis dreifach, gleich oder verschieden substituierten 1-Benzimidazolylrest oder 1-Indolylrest steht,

wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder jeweils gegebenenfalls im Phenylteil ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Benzyl, Phenoxy oder Benzyloxy und

$R^1$ und $R^2$ unabhängig voneinander jeweils für Methyl, Ethyl, n- oder i-Propyl, n-, i-, s- oder t-Butyl, für Allyl, Propargyl, Methoxymethyl, Methylthiomethyl, Fluorethyl, Chlorethyl, Bromethyl oder Trifluorethyl stehen.

Ganz besonders bevorzugt sind Verbindungen der Formel (I), bei welchen

A für einen gegebenenfalls ein- oder zweifach, gleich oder verschieden substituierten 1-Benzimidazolylrest oder 1-Indolylrest steht,

wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder jeweils gegebenenfalls ein- oder zweifach, gleich oder verschieden durch Fluor, Chlor, Brom, Methyl und/oder Ethyl substituiertes Phenyl, Phenoxy, Benzyl oder Benzyloxy und

$R^1$ und $R^2$ unabhängig voneinander jeweils für Methyl oder Ethyl stehen.

Insbesondere bevorzugt sind Verbindungen der Formel (I), bei welchen

A für einen gegebenenfalls in 6-Position substituierten 1-Indolylrest steht,

wobei als Substituenten infrage kommen: Fluor, Chlor, Brom, Cyano, Nitro, Methyl, Ethyl, Methoxy, Ethoxy, Methylthio, Trifluormethyl, Trifluormethoxy, Trifluormethylthio, Methoxycarbonyl, Ethoxycarbonyl, Methoximinomethyl, Methoximinoethyl oder jeweils gegebenenfalls ein-oder zweifach, gleich oder verschieden durch Fluor, Chlor, Brom oder Methyl substituiertes Phenyl, Phenoxy, Benzyl oder Benzyloxy und

$R^1$ und $R^2$ jeweils für Methyl stehen.

Im einzelnen seien außer den bei den Herstellungsbeispielen genannten Verbindungen die folgenden heterocyclisch substituierten Acrylsäureester der allgemeinen Formel (I) genannt:

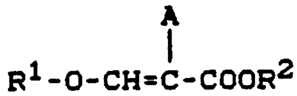

(I)

| $R^1$ | $R^2$ | A |
|---|---|---|
| $CH_3$ | $CH_3$ | 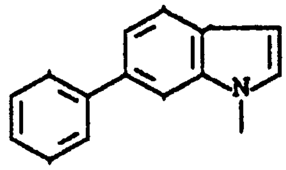 |
| $CH_3$ | $CH_3$ | 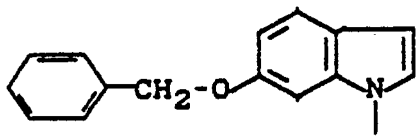 |
| $CH_3$ | $CH_3$ | 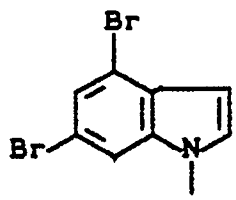 |

| $R^1$ | $R^2$ | A |
| --- | --- | --- |
| $CH_3$ | $CH_3$ | 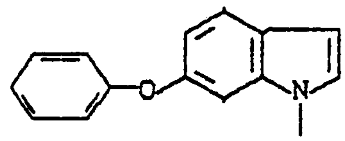 |
| $C_2H_5$ | $CH_3$ | 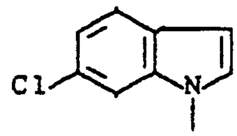 |
| $CH_3$ | $CH_3$ | 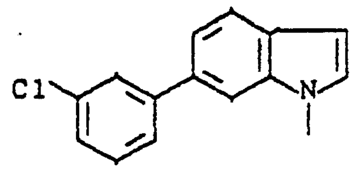 |
| $CH_3$ | $CH_3$ | 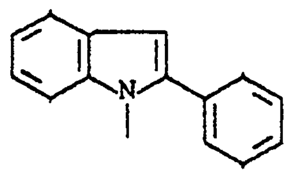 |
| $CH_3$ | $C_2H_5$ | 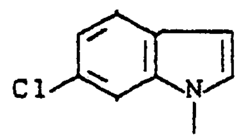 |
| $CH_3$ | $CH_3$ | 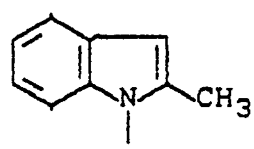 |
| $CH_3$ | $CH_3$ | 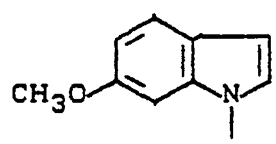 |
| $CH_3$ | $CH_3$ | 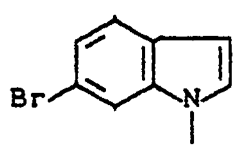 |

| $R^1$ | $R^2$ | A |
|---|---|---|
| $CH_3$ | $CH_3$ | 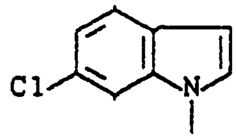 |
| $CH_3$ | $CH_3$ | 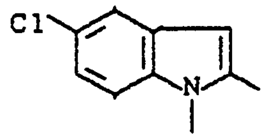 |
| $CH_3$ | $CH_3$ | 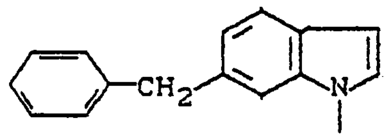 |
| $CH_3$ | $CH_3$ | 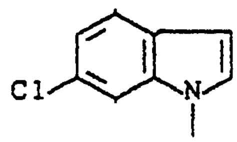 |
| $C_2H_5$ | $CH_3$ | 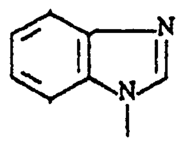 |
| $CH_3$ | $C_2H_5$ | 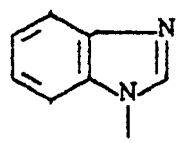 |
| $CH_3$ | $CH_3$ | 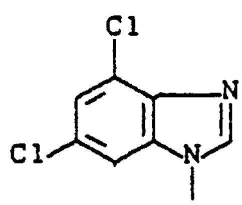 |
| $C_2H_5$ | $C_2H_5$ | 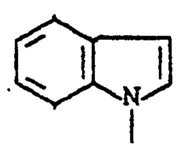 |

| $R^1$ | $R^2$ | A |
|---|---|---|
| $C_2H_5$ | $CH_3$ | 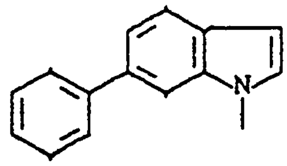 |
| $CH_3$ | $C_2H_5$ | 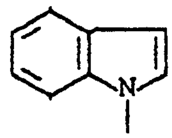 |
| $CH_3$ | $CH_3$ | 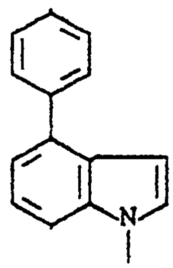 |
| $CH_3$ | $CH_3$ | 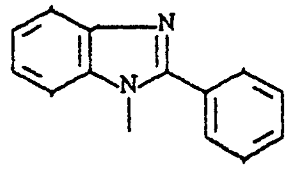 |
| $CH_3$ | $CH_3$ | 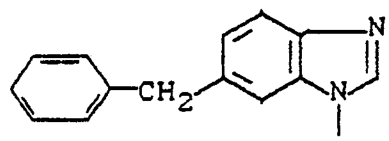 |
| $CH_3$ | $CH_3$ | 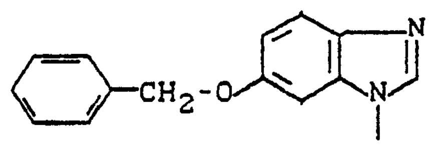 |
| $CH_3$ | $CH_3$ | 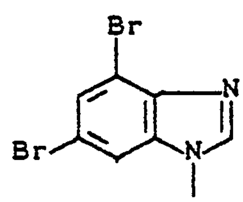 |

| $R^1$ | $R^2$ | A |
|---|---|---|
| $CH_3$ | $CH_3$ | 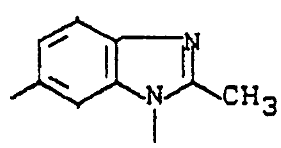 |

Verwendet man beispielsweise 3-Hydroxy-2-(6-chlorindol-1-yl)-acrylsäuremethylester und Dimethylsulfat

als Ausgangsstoffe, so läßt sich der Reaktionsablauf des erfindungsgemäßen Verfahrens (a) durch das folgende Formelschema darstellen:

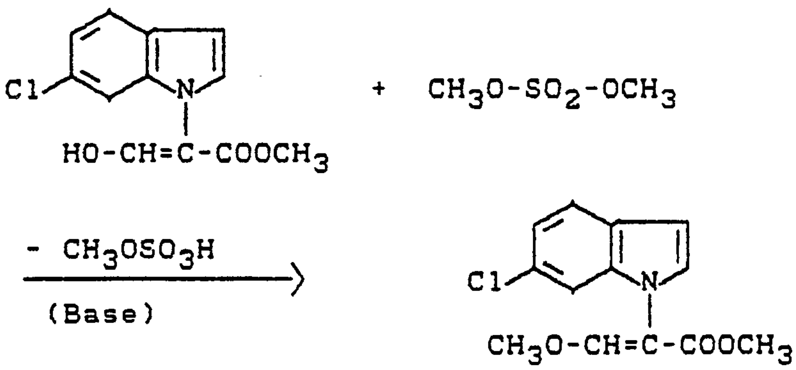


Verwendet man beispielsweise 2-(Indol-1-yl)-essigsäuremethylester, Ameisensäuremethylester und Diethylsulfat als Ausgangsstoffe, so läßt sich der Reaktionsablauf des erfindungsgemäßen Verfahrens (b) durch das folgende Formelschema darstellen:

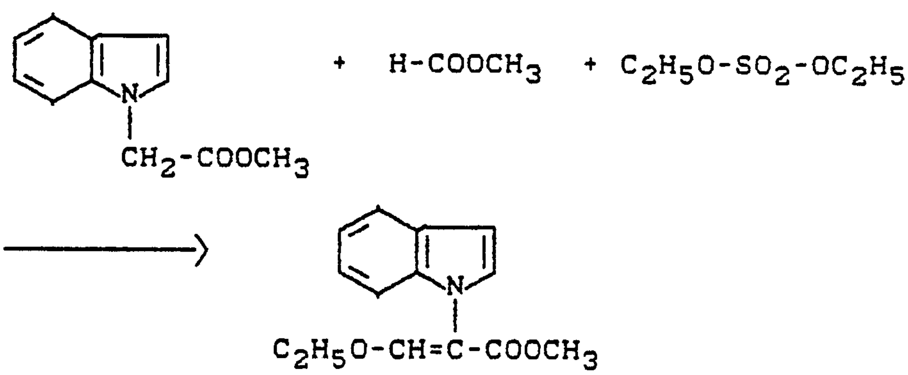

Die zur Durchführung des erfindungsgemäßen Verfahrens (a) als Ausgangsstoffe benötigten 3-Hydroxyacrylsäureester sowie deren Alkalimetallsalze sind durch die Formel (II) allgemein definiert. In dieser Formel (II) stehen A und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

M steht vorzugsweise für Wasserstoff oder für ein Alkalimetallkation, insbesondere für Wasserstoff, für ein Natrium- oder für ein Kaliumkation.

Die 3-Hydroxyacrylsäureester und deren Alkalimetallsalze der Formel (II) sind teilweise bekannt (vgl. J. org. Chem. 30, 1118 - 1122 [1965]). Noch nicht bekannt und ebenfalls Gegenstand der Erfindung sind 3-Hydroxyacrylsäureester und deren Alkalimetallsalze der Formel (IIa),

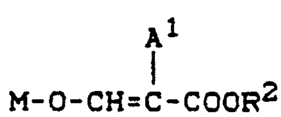
(IIa)

in welcher
$A^1$ für einen gegebenenfalls substituierten 1-Indolylrest steht und
$R^2$ und M die oben angegebene Bedeutung haben,
wobei als Substituenten für den Rest $A^1$ vorzugsweise diejenigen Reste infrage kommen, die bereits im Zusammenhang mit der Beschreibung der erfindungsgemäßen Stoffe der Formel (I) als bevorzugt für den Rest A genannt wurden. Für $R^2$ und M gelten als bevorzugt, besonders bevorzugt, ganz besonders und insbesondere bevorzugt die Definitionen, die bei der Beschreibung der erfindungsgemäßen Stoffe der

Formel (I) als bevorzugt für diese Substituenten genannt wurden.

Man erhält sie in Analogie zu bekannten Verfahren (vgl. z.B. J. org. Chem. 30, 1118-1122 [1965]), wenn man Indolylessigsäureester der Formel (IVa),

$A^1$-$CH_2$-$COOR^2$ (IVa)

in welcher

$A^1$ und $R^2$ die oben angegebene Bedeutung haben,

mit Ameisensäureestern der Formel (V),

$$R^3\text{-O-}\overset{\overset{\displaystyle O}{\|}}{C}\text{-H} \qquad (V)$$

in welcher

$R^3$ für Alkyl steht,

gegebenenfalls in Gegenwart eines Verdünnungsmittels wie beispielsweise Dimethylformamid und gegebenenfalls in Gegenwart eines basischen Reaktionshilfsmittels wie beispielweise Natriumhydrid bei Temperaturen zwischen - 20 °C und + 50 °C umsetzt.

Die zur Durchführung der erfindungsgemäßen Verfahren (a) und (b) weiterhin als Ausgangsstoffe benötigten Alkylierungsmittel sind durch die Formel (III) allgemein definiert. In dieser Formel (III) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

E steht für eine bei Alkylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest..

Die Alkylierungsmittel der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten heterocyclisch substituierten Essigsäureester sind durch die Formel (IV) allgemein definiert. In dieser Formel (IV) stehen A und $R^2$ vor zugsweise für diejenigen Reste, die bereits im Zusammenhang mit der Beschreibung der erfindungsgemäßen Stoffe der Formel (I) als bevorzugt für diese Substituenten genannt wurden.

Die heterocyclisch substituierten Essigsäureester der Formel (IV) sind bekannte Verbindungen oder erhältlich in Analogie zu bekannten Verfahren (vgl. z.B. J. Org. Chem. 30, 1118-1122 [1965]; DE-OS 19 01 167; DE-OS 19 08 541; DE-OS 27 01 095; DE-OS 27 02 462; US-PS 40 21 448; J. Amer. chem. Soc. 103, 7620-7629 [1981]), beispielsweise wenn mann 1H-Heterocyclen der Formel (VI),

A-H (VI)

in welcher

A die oben angegebene Bedeutung hat,

mit 2-Halogenessigsäureestern der Formel (VII),

X-$CH_2$-$COOR^2$ (VII)

in welcher

$R^2$ die oben angegebene Bedeutung hat und

X für Halogen, insbesondere für Brom steht,

gegebenenfalls in Gegenwart eines Verdünnungsmittels wie beispielsweise Dimethylformamid und gegebenenfalls in Gegenwart eines Reaktionshilfsmittels wie beispielsweise Natriumhydrid bei Temperaturen zwischen - 20 °C und + 60 °C umsetzt.

1H-Heterocyclen der Formel (VI) und 2-Halogenessigsäureester der Formel (VII) sind allgemein bekannte Verbindungen der organischen Chemie.

Die zur Durchführung des erfindungsgemäßen Verfahrens (b) und zur Synthese der Vorprodukte der Formel (II) weiterhin als Ausgangsstoffe benötigten Ameisensäureester sind durch die Formel (V) allgemein definiert. In dieser Formel (V) steht $R^3$ vorzugsweise für geradkettiges oder verzweigtes Alkyl mit 1 bis 4 Kohlenstoffatomen, insbesondere für Methyl oder Ethyl.

Die Ameisensäureester der Formel (V) sind ebenfalls allgemein bekannte Verbindungen der organischen Chemie.

Als Verdünnungsmittel zur Durchführung der erfindungsgemäßen Verfahren (a) und (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethyl-phosphorsäuretriamid oder Sulfoxide, wie Dimethylsulfoxid.

Das erfindungsgemäße Verfahren (a) kann gegebenenfalls auch in einem Zweiphasensystem, wie

beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyldimethyl-ammoniummethylsulfat, Dimethyl-$C_{12}/C_{14}$-alkylbenzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Die erfindungsgemäßen Verfahren (a) und (b) werden vorzugsweise in Gegenwart eines geeigneten basischen Reaktionshilfsmittels durchgeführt. Als solche kommen alle üblicherweise verwendbaren anorganischen und organischen Basen in Frage, Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (a) und (b) in einem größeren Bereich variiert werden. Im allgemeinen arbeitet man bei Temperaturen zwischen - 30 °C und + 120 °C, vorzugsweise bei Temperaturen zwischen 0 °C und 50 °C.

Zur Durchführung des erfindungsgemäßen Verfahrens (a) setzt man pro Mol an 3-Hydroxyacrylsäureester oder eines entsprechenden Alkalimetallsalzes der Formel (II) im allgemeinen 1.0 bis 2.0 Mol, vorzugsweise 1.0 bis 1.2 Mol an Alkylierungsmittel der Formel (III) und gegebenenfalls 1.0 bis 5.0 Mol, vorzugsweise 1.0 bis 2.0 Mol an Reaktionshilfsmittel ein.
Die Reaktionsdurchführung, Aufarbeitung und Isolierung der Reaktionsprodukte erfolgt nach allgemein üblichen Methoden (vgl. auch die Herstellungsbeispiele).

Zur Durchführung des erfindungsgemäßen Verfahrens (b) setzt man pro Mol an heterocyclisch substituierter Essigsäure der Formel (IV) im allgemeinen 1.0 bis 10.0 Mol, vorzugsweise 1.0 bis 5.0 Mol an Ameisensäureester der Formel (V) und 1.0 bis 1.5 Mol, vorzugsweise 1.0 bis 1.2 Mol an Alkylierungsmittel der Formel (III) sowie gegebenenfalls 1.0 bis 5.0 Mol, vorzugsweise 1.0 bis 2.0 Mol an Reaktionshilfsmittel ein.

Die Reaktionsdurchführung, Aufarbeitung und Isolierung der Reaktionsprodukte erfolgt nach allgemein üblichen Methoden (vgl. z.B. EP 206 523 sowie die Herstellungsbeispiele).

Die erfindungsgemäßen Wirkstoffe eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;

Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des Getreidebraunrostes (Puccinia recondita) oder zur Bekämpfung von Reiskrankheiten, wie beispielsweise gegen den Erreger der Reisfleckenkrankheit (Pyricularia oryzae) oder zur Bekämpfung von Krankheiten im Obstbau, wie beispielsweise gegen den Erreger des Apfelschorfes (Venturia inaequalis) einsetzen.

Darüberhinaus zeigen die erfindungsgemäßen Wirkstoffe eine breite in-vitro-Wirksamkeit.

In entsprechenden Aufwandmengen zeigen die erfindungsgemäßen Wirkstoffe außerdem auch akarizide, nematizide und herbizide Eigenschaften und lassen sich beispielsweise als selektive Herbizide in Reiskulturen einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkei ten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizine und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%. vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1

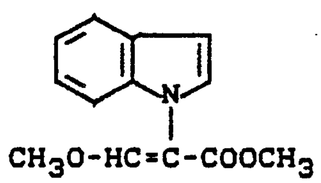

**(Verfahren a)**

Zu einer Suspension von 6,6 g (0,0478 Mol) Kaliumcarbonat in 50 ml Dimethylformamid gibt man bei Raumtemperatur tropfenweise unter Rühren 5,2 g (0.0240 Mol) 3-Formyl-2-(1-indolyl)-essigsäureethylester in 20 ml Dimethylformamid und anschließend ebenfalls tropfenweise unter Rühren 3,0 g (0.0238 Mol) Dimethylsulfat und rührt nach beendeter Zugabe weitere 45 Minuten bei Raumtemperatur.

Zur Aufarbeitung gießt man die Reaktionsmischung in eine gesättigte wässrige Natriumhydrogencarbonatlösung, extrahiert mehrfach mit Ether, wäscht die vereinigten organischen Phasen mit Wasser, trocknet über Natriumsulfat, engt im Vakuum ein und kristallisiert den Rückstand aus Methanol um.

Man erhält 2,9 g (52 % der Theorie) an (Z)-3-Methoxy-2-(indol-1-yl)-acrylsäuremethylester vom Schmelzpunkt 117 °C.

Herstellung der Ausgangsverbindung

Beispiel II-1

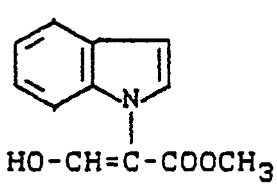

Zu einer Suspension von 2,41 g (0.0803 Mol) Natriumhydrid (80prozentig in Mineralöl) in 50 ml Dimethylformamid gibt man bei Raumtemperatur tropfenweise unter Rühren eine Lösung von 7,6 g (0.0402 Mol) 2-(Indol-1-yl)-essigsäuremethylester und 23,9 g (0.3980 Mol) Ameisensäuremethylester in 40 ml Dimethylformamid. Nach beendeter Zugabe rührt man weitere 2 Stunden bei Raumtemperatur, gießt dann

die Mischung in Eiswasser, stellt mit Natriumcarbonat alkalisch, wäscht zweimal mit Ether, säuert dann mit konzentrierter Salzsäure an, extrahiert mehrfach mit Ether, trocknet die vereinigten Etherphasen über Natriumsulfat, engt im Vakuum ein und reinigt den Rückstand durch Säulenchromatographie (Kieselgel; Laufmittel: Dichlormethan).

Man erhält 5,3 g (61 % der Theorie) an 3-Hydroxy-2-(indol-1-yl)-acrylsäuremethylester als Öl.
$^1$H-NMR ($CDCl_3$) : $\delta$ = 8,4 ppm.

Beispiel 2

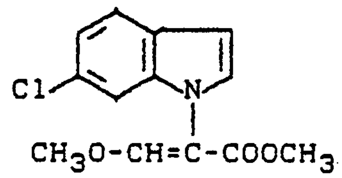

(Verfahren b)

Zu einer Suspension von 1,92 g (0.064 Mol) Natriumhydrid (80prozentig in Mineralöl) in 30 ml absolutem Dimethylformamid gibt man tropfenweise unter Rühren und Kühlung 7,2 g (0.032 Mol) 2-(6-Chlorindol-1-yl)-essigsäuremethylester so zu, daß die Temperatur der Reaktionsmischung 5 °C nicht übersteigt. Nach beendeter Zugabe rührt man weitere 15 Minuten bei 0 °C bis 5 °C, tropft 9,6 g (0.16 Mol) Ameisensäuremethylester zu und läßt dann den Ansatz langsam auf Raumtemperatur kommen, wobei eine heftige Gasentwicklung auftritt. Nach 2 Stunden wird wiederum auf 5 °C abgekühlt, 8,06 g (0.064 Mol) Dimethylsulfat zugetropft und anschließend 18 Stunden bei Raumtemperatur gerührt.
Zur Aufarbeitung gießt man die Reaktionsmischung in Eiswasser, extrahiert mehrfach mit Ether, wäscht die vereinigten organischen Phasen mit Wasser, trocknet über Natriumsulfat, engt im Vakuum ein und chromatographiert den öligen Rückstand an Kieselgel (Laufmittel: Dichlormethan).

Man erhält zunächst als 1. Fraktion 4,7 g (55 % der Theorie) and (Z)-3-Methoxy-2-(6-chlorindol-1-yl)-acrylsäuremethylester als Öl
$^1$H-NMR ($CDCl_3$) : $\delta$ = 3,7; 3,9 ppm
und als 2. Fraktion 1,0 g (12 % der Theorie) an (E)-3-Methoxy-2-(6-chlorindol-1-yl)-acrylsäuremethylester als Öl
$^1$H-NMR ($CDCl_3$) : $\delta$ = 3,7; 4,05 ppm.

Herstellung der Ausgangsverbindung

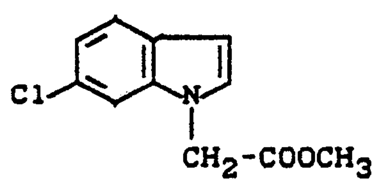

5,0 g (0.033 Mol) 6-Chlorindol, 6,88 g (0.045 Mol) Bromessigsäuremethylester und 4,5 g (0.033 Mol) Kaliumcarbonat in 100 ml Aceton werden 4 Stunden auf Rückflußtemperatur erhitzt, anschließend mit weiteren 2,0 g (0.0144 Mol) Kaliumcarbonat und 3,0 g (0,0196 Mol) Bromessigsäuremethylester versetzt und weitere 4 Stunden auf Rückflußtemperatur erhitzt. Zur Aufarbeitung wird die Mischung im Vakuum eingeengt, der Rückstand in Dichlormethan aufgenommen, mehrfach mit Wasser gewaschen, über Natriumsulfat getrocknet und im Vakuum eingeengt.

Man erhält 7,2 g (100 % der Theorie) an 2-(6-Chlorindol-1-yl)-essigsäuremethylester als Öl
$^1$H-NMR ($CDCl_3$: $\delta$ =. 3,7; 4,8 ppm.

In entsprechender Weise und gemäß den allgemeinen Angaben zur Herstellung erhält man die

folgenden heterocyclisch substituierten Acrylsäureester der allgemeinen Formel (I):

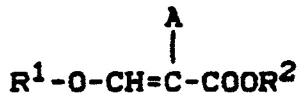 (I)

| Bsp. Nr. | $R^1$ | $R^2$ | A | physikalische Eigenschaften |
|---|---|---|---|---|
| 3 | $CH_3$ | $CH_3$ | 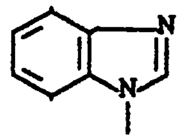 | $^1$H-NMR*): 3,75; 3,9 |
| 4 | $CH_3$ | $CH_3$ | 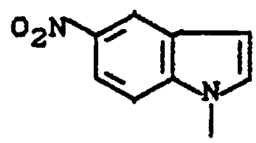 | $^1$H-NMR*): 3,7; 4,05 (Z-Form) |
| 5 | $CH_3$ | $CH_3$ | 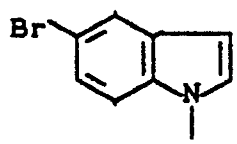 | $^1$H-NMR*): 3,65; 4,05 (Z-Form) |
| 6 | $CH_3$ | $CH_3$ | 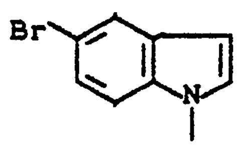 | $^1$H-NMR*): 3,75; 3,9 (E-Form) |
| 7 | $CH_3$ | $CH_3$ | 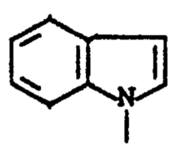 | Fp. 113° C (E-Form) |
| 8 | $CH_3$ | $CH_3$ | 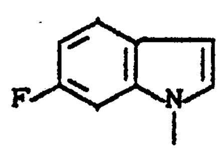 | Fp. 117-119° C (Z-Form) |
| 9 | $CH_3$ | $CH_3$ | 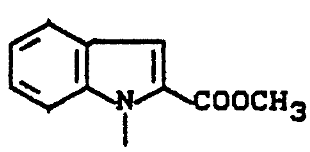 | Fp. 132° C (Z-Form) |

| Bsp. Nr. | $R^1$ | $R^2$ | A | physikalische Eigenschaften |
|---|---|---|---|---|
| 10 | $CH_3$ | $CH_3$ | 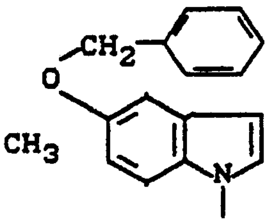 | Fp. 151-154° C (Z-Form) |
| 11 | $CH_3$ | $CH_3$ | 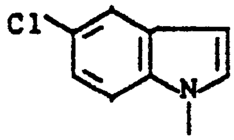 | $^1$H-NMR*): 3,72; 3,82 (Z-Form) |
| 12 | $CH_3$ | $CH_3$ | 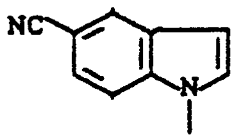 | $^1$H-NMR*): 3,75; 3,9 (Z-Form) |
| 13 | $CH_3$ | $CH_3$ | 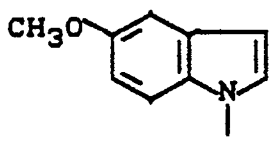 | $^1$H-NMR*): 3,72; 3,84 (Z-Form) |
| 14 | $CH_3$ | $CH_3$ | 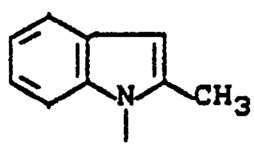 | $^1$H-NMR*): 3,68; 3,83 (Z-Form) |
| 15 | $CH_3$ | $CH_3$ | 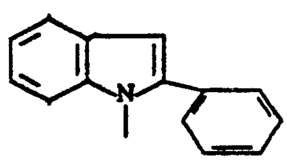 | $^1$H-NMR*): 3,57; 3,78 (Z-Form) |
| 16 | $CH_3$ | $CH_3$ | 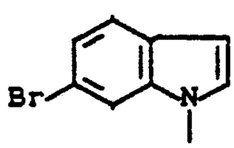 | Fp. 129° C (Z-Form) |

| Bsp. Nr. | $R^1$ | $R^2$ | A | physikalische Eigenschaften |
|---|---|---|---|---|
| 17 | $CH_3$ | $CH_3$ | 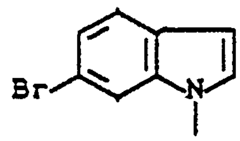 | Fp. 162°C (E-Form) |
| 18 | $CH_3$ | $CH_3$ | 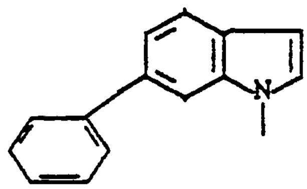 | $^1$H-NMR*): 3,75; 3,89 (Z-Form) |
| 19 | $CH_3$ | $CH_3$ | 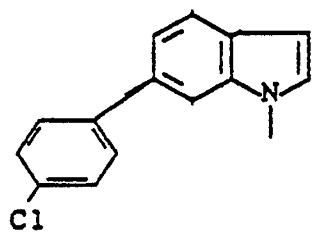 | $^1$H-NMR*): 3,73; 3,88 |
| 20 | $CH_3$ | $CH_3$ | 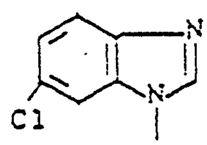 | |
| 21 | $CH_3$ | $CH_3$ | 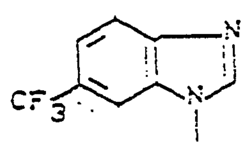 | |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

## Anwendungsbeispiele

In dem folgenden Anwendungsbeispiel wurde die nachstehend aufgeführte Verbindung als Vergleichsubstanz eingesetzt:

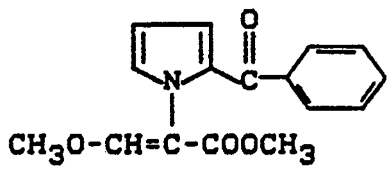

(A)

3-Methoxy-2-(2-benzoylpyrrol-1-yl)-acrylsäuremethylester
(bekannt aus EP 206 523).

Beispiel A

Puccinia-Test (Weizen) / protektiv

Lösungsmittel: 100 Gewichtsteile Dimethylformamid
Emulgator: 0,025 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigt bei diesem Test z.B. die Verbindung gemäß Herstellungsbeispiel 2.

**Ansprüche**

1. Heterocyclisch substituierte Acrylsäureester der allgemeinen Formel (I),

$$R^1\text{-O-CH} = \overset{\overset{A}{|}}{C}\text{-COOR}^2 \qquad (I)$$

in welcher
A für einen gegebenenfalls substituierten 1-Benzimidazolylrest oder für einen gegebenenfalls substituierten 1-Indolylrest steht und
$R^1$ und $R^2$ unabhängig voneinander jeweils für Alkyl, Alkenyl, Alkinyl, Halogenalkyl, Alkoxyalkyl oder Alkylthioalkyl stehen, deren geometrische Isomere und Isomerengemische.

2. Heterocyclisch substituierte Acrylsäureester gemäß Anspruch 1, wobei in der Formel (I)
A für einen jeweils gegebenenfalls einfach oder mehrfach, gleich oder verschieden substituierten 1-Benzimidazolylrest oder 1-Indolylrest steht, wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen oder jeweils gegebenenfalls im Arylteil einfach oder mehrfach, gleich oder verschieden durch Halogen, Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil und
$R^1$ und $R^2$ unabhängig voneinander jeweils für jeweils geradkettiges oder verzweigtes Alkyl mit 1 bis 6 Kohlenstoffatomen, Alkenyl mit 2 bis 6 Kohlenstoffatomen, Alkinyl mit 2 bis 6 Kohlenstoffatomen, Alkoxyalkyl oder Alkylthioalkyl mit jeweils 1 bis 4 Kohlenstoffatomen in den einzelnen Alkylteilen oder Halogenalkyl mit 1 bis 4 Kohlenstoffatomen und 1 bis 9 gleichen oder verschiedenen Halogenatomen stehen, deren geometrische Isomere und Isomerengemische.

3. Heterocyclisch substituierte Acrylsäureester gemäß Anspruch 1, wobei in der Formel (I) A für einen jeweils gegebenenfalls ein- bis dreifach, gleich oder verschieden substituierten 1-Benzimidazolylrest oder 1-Indolylrest steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder jeweils gegebenenfalls im Phenylteil ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Benzyl, Phenoxy oder Benzyloxy und
$R^1$ und $R^2$ unabhängig voneinander jeweils für Methyl, Ethyl, n- oder i-Propyl, n-, i-, s-oder t-Butyl, für Allyl, Propargyl, Methoxymethyl, Methylthiomethyl, Fluorethyl, Chlorethyl, Bromethyl oder Trifluorethyl stehen, deren geometrische Isomere und Isomerengemische.

4. Heterocyclisch substituierte Acrylsäureester gemäß Anspruch 1, wobei in der Formel (I) A für einen gegebenenfalls ein- oder zweifach, gleich oder verschieden substituierten 1-Benzimidazolylrest oder 1-Indolylrest steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder jeweils gegebenenfalls ein- oder zweifach, gleich oder verschieden durch Fluor, Chlor, Brom, Methyl und/oder Ethyl substituiertes Phenyl, Phenoxy, Benzyl oder Benzyloxy und
$R^1$ und $R^2$ unabhängig voneinander jeweils für Methyl oder Ethyl stehen, deren geometrische Isomere und Isomerengemische.

5. Heterocyclisch substituierte Acrylsäureester gemäß Anspruch 1, wobei in der Formel (I) A für einen gegebenenfalls in 6-Position substituierten 1-Indolylrest steht, wobei als Substituenten infrage kommen: Fluor, Chlor, Brom, Cyano, Nitro, Methyl, Ethyl, Methoxy, Ethoxy, Methylthio, Trifluormethyl, Trifluormethoxy, Trifluormethylthio, Methoxycarbonyl, Ethoxycarbonyl, Methoximinomethyl, Methoximinoethyl oder jeweils gegebenenfalls ein- oder zweifach, gleich oder verschieden durch Fluor, Chlor, Brom oder Methyl substituiertes Phenyl, Phenoxy, Benzyl oder Benzyloxy und
$R^1$ und $R^2$ jeweils für Methyl stehen, deren geometrische Isomere und Isomerengemische.

6. Verfahren zur Herstellung von heterocyclisch substituierten Acrylsäureestern der allgemeinen Formel (I),

$$R^1\text{-O-CH}=\overset{\overset{\displaystyle A}{|}}{C}\text{-COOR}^2 \quad (I)$$

in welcher
A für einen gegebenenfalls substituierten 1-Benzimidazolyl oder für einen gegebenenfalls substituierten 1-Indolylrest steht und
$R^1$ und $R^2$ unabhängig voneinander jeweils für Alkyl, Alkenyl, Alkinyl, Halogenalkyl, Alkoxyalkyl oder Alkylthioalkyl stehen,
dadurch gekennzeichnet, daß man

(a) 3-Hydroxyacrylsäureester oder deren Alkalimetallsalze der Formel (II),

$$\text{M-O-CH}=\overset{\overset{\displaystyle A}{|}}{C}\text{-COOR}^2 \quad (II)$$

in welcher
A und $R^2$ die oben angegebene Bedeutung haben und
M für Wasserstoff oder für ein Alkalimetallkation steht,
mit Alkylierungsmitteln der Formel (III),

$$R^1\text{-E} \quad (III)$$

in welcher
$R^1$ die oben angegebene Bedeutung hat und
E für eine elektronenanziehende Abgangsgruppe steht,
gegebenenfalls in Gegenwart eines Verdünnungsmittels und gegebenenfalls in Gegenwart eines Reaktionshilfsmittels umsetzt oder daß man

(b) heterocyclisch substituierte Essigsäureester der Formel (IV),

$$\text{A-CH}_2\text{-COOR}^2 \quad (IV)$$

in welcher
A und $R^2$ die oben angegebene Bedeutung haben,

mit Ameisensäureestern der Formel (V),

O
‖
$R^3$-O- C -H (V)

in welcher
$R^3$ für Alkyl steht,
und anschließend mit Alkylierungsmitteln der Formel (III),
$R^1$-E (III)
in welcher
$R^1$ die oben angegebene Bedeutung hat und
E für eine elektronenanziehende Abgangsgruppe steht,
gegebenenfalls in Gegenwart eines Verdünnungsmittels und gegebenenfalls in Gegenwart eines Reaktionshilfsmittels umsetzt.

7. Schädlingsbekämpfungsmittel, gekennzeichnet durch einen Gehalt an mindestens einem heterocyclisch substituierten Acrylsäureester der Formel (I) nach den Ansprüchen 1 oder 6.

8. Verwendung von heterocyclisch substituierten Acrylsäureestern der Formel (I) nach den Ansprüchen 1 oder 6 zur Bekämpfung von Schädlingen.

9. Verfahren zur Bekämpfung von Schädlingen, dadurch gekennzeichnet, daß man heterocyclisch substituierte Acrylsäureester der Formel (I) nach den Ansprüchen 1 oder 6 auf Schädlinge und/oder ihren Lebensraum einwirken läßt.

10. Verfahren zur Herstellung von Schädlingsbekämpfungsmitteln, dadurch gekennzeichnet, daß man heterocyclisch substituierte Acrylsäureester der Formel (I) nach den Ansprüchen 1 oder 6 mit Streckmitteln und/oder oberflächenaktiven Mitteln vermischt.

11. 3-Hydroxyacrylsäureester der Formel (IIa),

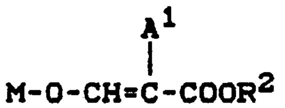

(IIa)

in welcher
$A^1$ für einen gegebenenfalls substituierten 1-Indolylrest steht,
$R^2$ für Alkyl, Alkenyl, Alkinyl, Halogenalkyl, Alkoxyalkyl oder Alkylthioalkyl steht und
M für Wasserstoff oder für ein Alkalimetallkation steht.

12. Verfahren zur Herstellung von 3-Hydroxyacrylsäureestern der Formel (IIa),

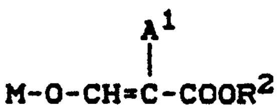

(IIa)

in welcher
$A^1$ für einen gegebenenfalls substituierten 1-Indolylrest steht,
$R^2$ für Alkyl, Alkenyl, Alkinyl, Halogenalkyl, Alkoxyalkyl oder Alkylthioalkyl steht und
M für Wasserstoff oder für ein Alkalimetallkation steht,
dadurch gekennzeichnet, daß man Indolylessigsäureester der Formel (IVa),
$A^1-CH_2-COOR^2$ (IVa)
in welcher
$A^1$ und $R^2$ die oben angegebene Bedeutung haben,
mit Ameisensäureestern der Formel (V),

O
‖
$R^3$-O- C -H (V)

in welcher
$R^3$ für Alkyl steht,
gegebenenfalls in Gegenwart eines Verdünnungsmittels und gegebenenfalls in Gegenwart eines basischen Reaktionshilfsmittels bei Temperaturen zwischen - 20 °C und + 50 °C umsetzt.

Europäisches Patentamt

# EUROPÄISCHER RECHERCHENBERICHT

Nummer der Anmeldung

EP 89 10 2204

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP-A-0 274 825 (ICI)<br>* Ansprüche 1,5 *<br>--- | 1,8 | C 07 D 209/08<br>C 07 D 235/08<br>A 01 N 43/38<br>A 01 N 43/52 |
| A | DE-A-1 803 728 (CIBA-GEIGY)<br>* Anspruch 1 *<br>--- | 1,8 | |
| A,D | EP-A-0 206 523 (ICI)<br>* Ansprüche 1,11 *<br>----- | 1,8 | |
| | | | **RECHERCHIERTE SACHGEBIETE (Int. Cl.4)** |
| | | | C 07 D 209/00<br>C 07 D 235/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 17-05-1989 | CASADO Y MARTIN DE MERCA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.....................................................................
& : Mitglied der gleichen Patentfamilie, übereinstimmendes Dokument

EPO FORM 1503 03.82 (P0403)